# EUROPEAN PATENT APPLICATION

(11) **EP 3 315 071 A1**
(43) Date of publication of application: **02.05.2018**
(21) Application number: 16830219.8
(22) Date of filing: 29.06.2016
(51) Int. Cl.: A61B 5/1455

(54) **SENSOR MODULE AND BIOMETRIC INFORMATION DISPLAY SYSTEM**

(30) Priority: 30.07.2015 JP 2015150600
(71) Applicant: Alps Electric Co., Ltd., Tokyo 145-8501 (JP); Genial Light Co., Ltd., Shizuoka 430-0917 (JP)
(72) Inventor: OTAKI, Yukio, Tokyo 145-8501 (JP); YOSHIMURA, Takashi, Tokyo 145-8501 (JP); SAKURAI, Masaru, Tokyo 145-8501 (JP); CHAEN, Katsuyoshi, Tokyo 145-8501 (JP); SOETA, Kaoru, Tokyo 145-8501 (JP); SHIMOKITA, Ryo, Hamamatsu-shi Shizuoka 430-0917 (JP)
(74) Representative: Schmitt-Nilson Schraud Waibel Wohlfrom Patentanwälte Partnerschaft mbB
(86) International application number: PCT/JP2016/069207
(87) International publication number: WO 2017/018114

(57) **Abstract**

For a sensor module that is easily positioned at a position at which biological information can be obtained when attached to an object under detection and a biological information displaying system that includes the sensor module, provided is a biological information displaying system 1 that includes a sensor module 10 that includes a light emitting part 11 including a light emitting element 11a1 and a light emitting element 11a2 arranged at an interval; and a light receiving part 12 including one light receiving element 12-1 sensitive to near-infrared light. The one light receiving element 12-1 is arranged to receive light emitted by the light emitting element 11a1 and the light emitting element 11b1, and the light emitting element 11a1 and the light emitting element 11b1 emit near-infrared light having a same central wavelength λ1.

## Description

### TECHNICAL FIELD

The present invention relates to a sensor module for estimating biological information that emits light including near-infrared light to an object under detection, receives light that has passed through the object under detection, and generates a signal in accordance with near-infrared light included in the received light, and relates to a biological information displaying system that includes the sensor module.

### BACKGROUND ART

A pulse wave sensor disclosed in Patent Document 1 includes a sensor part including a pair of a light emitting element and a light receiving element arranged on the back surface side of a transparent plate to be brought into contact with a wrist of an object under detection. Near infrared light emitted from the light emitting element in the wrist direction is reflected by red blood cells flowing through an artery of the wrist, and the reflected light is detected by the light receiving element to detect the pulse wave of the object under detection.

### RELATED-ART DOCUMENTS

### Patent Documents

Patent Document 1: Japanese Laid-Open Patent Publication No. 2004-275307

### DISCLOSURE OF INVENTION

### PROBLEMS TO BE SOLVED BY THE INVENTION

With regard to the above, although a practical detection sensitivity is obtained by arranging the light emitting element and the light receiving element at appropriate positions with respect to an artery, there is a problem that it is difficult to perform a positional adjustment because a range satisfying such appropriate positions is narrow.

The present invention is made in view of the above described problem. An object of the present invention is to provide a sensor module that is easily positioned at a position at which biological information can be obtained when attached to an object under detection and to provide a biological information displaying system that includes the sensor module.

### MEANS FOR SOLVING THE PROBLEMS

In order to solve the above described problem, a first aspect of the present invention provides a sensor module for estimating biological information including: a light emitting part configured to emit light including near-infrared light to an object under detection; and a light receiving part configured to receive the light that has passed through the object under detection. The sensor module generates a signal in accordance with near-infrared light included in the light received by the light receiving part. The light emitting part includes a plurality of light emitting elements arranged at an interval. The light receiving part includes one or more light receiving elements that are sensitive to near-infrared light. The one or more light receiving elements are arranged to receive the light emitted by each of the plurality of light emitting elements. Each of the plurality of light emitting elements emits near-infrared light having a same central wavelength.

The light receiving elements may be arranged between two light emitting elements included in the plurality of light emitting elements. The light receiving elements may be arranged with the plurality of light emitting elements at an interval of from 4 mm to 11 mm. The light emitting part may further include a plurality of other light emitting elements each of which emits near-infrared light having another same central wavelength, which is different from the central wavelength of the near-infrared light emitted by the plurality of light emitting elements. Each of the plurality of light emitting elements may emit near-infrared light having the central wavelength and near-infrared light having another same central wavelength, which is different from the central wavelength. The central wavelength may be shorter than 805 nm, and said another central wavelength may be longer than 805 nm. The central wavelength may be 760 nm, and said another central wavelength may be 850 nm. The light emitting part may further include a plurality of other light emitting elements each of which emits near-infrared light having another same central wavelength, which is different from the central wavelength of the near-infrared light emitted by the plurality of light emitting elements, and said another central wavelength may be 640 nm, and the central wavelength may be 940 nm. A wavelength of light at which receiving sensitivity in the one or more light receiving elements is highest may be the central wavelength. The light receiving part may further include one or more other light receiving elements that are sensitive to near-infrared light, the one or more other light receiving elements may be arranged to receive the light emitted by each of the plurality of other light emitting elements, a wavelength of light at which receiving sensitivity in the one or more light receiving elements is highest may be the central wavelength, and a wavelength of light at which receiving sensitivity in the one or more other light receiving elements is highest may be said another central wavelength.

A second aspect of the present invention provides a biological information displaying system including: the above described sensor module; a biological information estimating part configured to estimate biological information based on the signal generated by the receiving part; and a display part configured to display the biological information estimated by the biological information estimating part. The biological information estimating part may estimate, as the biological information, at least one selected from a hemoglobin change in blood, an oxygen rate change in blood, and a pulse rate.

### ADVANTAGEOUS EFFECT OF THE INVENTION

According to the present invention, it is possible to position a sensor module at a position at which biological information can be obtained when attached to an object under detection.

### BRIEF DESCRIPTION OF THE DRAWINGS

FIG. 1 is a perspective view of a biological information displaying system according to an embodiment of the present invention.
FIG. 2 is a functional block diagram of the biological information displaying system of FIG. 1.
FIG. 3 is a perspective view of a sensor module included in the biological information displaying system of FIG. 1.
FIG. 4 is a planar view of a substrate unit included in the sensor module of FIG. 3.
FIG. 5 is a graph illustrating, for each of a configuration including two light emitting elements constituting a pair and a configuration including only one light emitting element, a relationship between detection sensitivity and a planar distance from a reactive member.
FIG. 6 is a graph illustrating a relationship between detection sensitivity and a planar distance from a reactive member for a configuration in which the interval between a light emitting element and a light receiving element is 2.5 mm.
FIG. 7 is a graph illustrating a relationship between detection sensitivity and a planar distance from a reactive member for a configuration in which the interval between a light emitting element and a light receiving element is 4 mm.
FIG. 8 is a graph illustrating a relationship between detection sensitivity and a planar distance from a reactive member for a configuration in which the interval between a light emitting element and a light receiving element is 7 mm.
FIG. 9 is a graph illustrating a relationship between detection sensitivity and a planar distance from a reactive member for a configuration in which the interval between a light emitting element and a light receiving element is 10 mm.

### DESCRIPTION OF EMBODIMENTS

In the following, embodiments of the present invention will be described with reference to the drawings. Note that the same reference numerals are given to the same members and descriptions for members already described may be omitted as appropriate. Also, descriptions of indicating "top and bottom" are used to conveniently describe a relative positional relationship between respective members, and are not to indicate an absolute positional relationship.

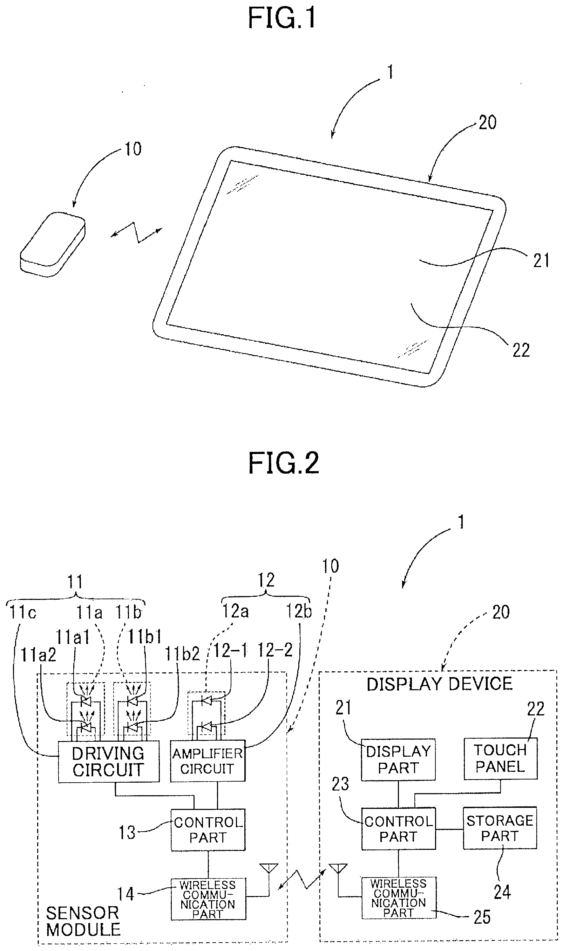

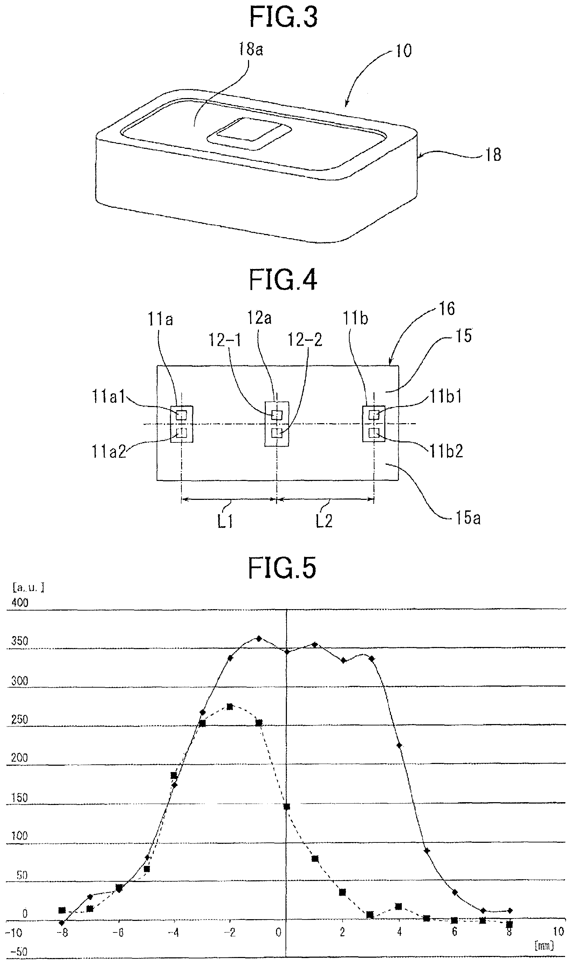

FIG. 1 is a perspective view of a biological information displaying system according to an embodiment of the present invention. FIG. 2 is a functional block diagram of the biological information displaying system of FIG. 1. FIG. 3 is a perspective view of a sensor module included in the biological information displaying system of FIG. 1. FIG. 4 is a planar view of a substrate unit included in the sensor module of FIG. 3.

The biological information displaying system 1 according to the present embodiment illustrated in FIG. 1 includes a portable sensor module 10, which is attached by a rubber band or the like to be directly in contact with an arm, a chest, or the like of an object under detection that is a biological object of a human, estimates biological information about the object under detection, and transmits the estimated biological information through wireless communication, and includes a display device 20 that displays the biological information that is transmitted from the sensor module 10.

As illustrated in FIG. 2 to FIG. 4, the sensor module 10 includes a light emitting part 11, a light receiving part 12, a control part 13, a wireless communication part 14, a substrate unit 16 that includes a substrate 15 on which these parts are mounted, and a case 18 that accommodates the substrate unit 16. Further, the sensor module 10 includes a non-illustrated power source circuit for realizing a battery operation.

The light emitting part 11 includes a light emitting element package 11a, a light emitting element package 11b, and a driving circuit 11c. The light emitting element package 11a contains, within one package, a light emitting element 11a1 and a light emitting element 11a2 made of elements such as light-emitting diode elements or laser elements that emit light including near-infrared light. Similarly, the light emitting element package 11b contains, within one package, a light emitting element 11b1 and a light emitting element 11b2 made of elements such as light-emitting diode elements or laser elements that emit light including near-infrared light. The driving circuit 11c drives the light emitting element 11a1 and the light emitting element 11a2, which are included in the light emitting element package 11a, and the light emitting element 11b1 and the light emitting element 11b2, which are included in the light emitting element package 11b.

The light emitting element 11a1 and the light emitting element 11b1 as "light emitting elements" constitute a pair. As each of the light emitting element 11a1 and the light emitting element 11b1, an element is used that is able to emit near-infrared light having the same wavelength, which is shorter than 805 nm, as a central wavelength λ1 as "a central wavelength". Also, the light emitting element 11a2 and the light emitting element 11b2 as "other light emitting elements" constitute a pair. As each of the light emitting element 11a2 and the light emitting element 11b2, an element is used that is able to emit near-infrared light having the same wavelength, which is longer than 805 nm, as a central wavelength λ2 as "another central wavelength". Note that 805 nm is a wavelengh that is less affected by water that accounts for a large portion of a living body as an object under detection, and using wavelengths around 805 nm to observe a difference of absorbances of hemoglobin into the body allows to estimate biological inforamation with high accuracy. Here, in the present specification, the central wavelength of near-infrared light refers to a wavelength at which the intensity of light (energy of light) is the highest within the wavelength range of near-infrared light emitted by a light emitting element.

According to the present embodiment, the light emitting element 11a1 and the light emitting element 11b1 are able to emit near-infrared light whose central wavelength λ1 is 760 nm, and the light emitting element 11a2 and the light emitting element 11b2 are able to emit near-infrared light whose central wavelength λ2 is 850 nm. Note that the central wavelengths are not limited to these. It is preferable that the central wavelength λ1 of near-infrared light emitted by the light emitting element 11a1 and the light emitting element 11b1 is shorter than 805 nm, and the central wavelength λ2 of near-infrared light emitted by the light emitting element 11a2 and the light emitting element 11b2 is longer than 805 nm. For example, the central wavelength λ1 may be 780 nm and the central wavelength λ2 may be 830 nm. Further, the other light emitting elements may be configured to emit light other than near-infrared light such as red light, the central wavelength λ1 may be 640 nm, and the central wavelength λ2 may be 940 nm. It is preferable that the wavelength range of near-infrared light emitted by the light emitting element 11a1 and the light emitting element 11b1 is 760 ± 50 nm, and the wavelength range of near-infrared light emitted by the light emitting element 11a2 and the light emitting element 11b2 is 850 ± 50 nm. It is more preferable that the wavelength range of near-infrared light emitted by the light emitting element 11a1 and the light emitting element 11b1 is 760 ± 20 nm, and the wavelength range of near-infrared light emitted by the light emitting element 11a2 and the light emitting element 11b2 is 850 ± 20 nm. Such a configuration can increase an output of the light receiving part 12 and increase the S/R ratio. Note that the light emitting element 11a2 and the light emitting element 11b2 may be omitted and each of the light emitting element 11a1 and the light emitting element 11b1 may be configured to emit both near-infrared light of the central wavelength λ1 and near-infrared light of the central wavelength X2 separately, for example. Note that the "omission" includes a case of physical removal and also includes a case of simply not exerting a function as an element (that is, not using).

The light receiving part 12 includes a light receiving element package 12a and an amplifier circuit 12b. The light receiving element package 12a contains, within one package, a light receiving element 12-1 and a light receiving element 12-2 that output signals (light reception signals) in accordance with received near-infrared light. The amplifier circuit 12b amplifies the light reception signals output by the light receiving element 12-1 and the light receiving element 12-2, which are included in the light receiving element package 12a.

The light receiving element 12-1 as "a light receiving element" has the maximum receiving sensitivity at the central wavelength λ1 of near-infrared light emitted by the light emitting element 11a1 and the light emitting element 11b1, and is sensitive to near-infrared light having wavelengths close to the central wavelength λ1. Also, the light receiving element 12-2 as "another light receiving element" has the maximum receiving sensitivity at the central wavelength λ2 of near-infrared light emitted by the light emitting element 11a2 and the light emitting element 11b2, and is sensitive to near-infrared light having wavelengths close to the central wavelength λ2. According to the present embodiment, the receiving sensitivity of the light receiving element 12-1 is the maximum at a wavelength of 760 nm, and the light receiving element 12-1 is able to receive near-infrared light having wavelengths in a range of from 760 ± 50 nm. Further, the receiving sensitivity of the light receiving element 12-2 is the maximum at a wavelength of 850 nm, and the light receiving element 12-2 is able to receive near-infrared light having wavelengths in a range of from 850 ± 50 nm. It is preferable to select light receiving elements whose receiving sensitivity becomes the highest in accordance with central wavelengths emitted by light emitting elements. Note that the light receiving element 12-2 may be omitted and the light receiving element 12-1 may be configured to be sensitive to both near-infrared light having wavelengths close to the central wavelength λ1 and near-infrared light having wavelengths close to the central wavelength λ2, for example.

In order to receive light emitted by the light emitting element 11a1 and the light emitting element 11b1, the light receiving element 12-1 is arranged, on an upper surface 15a of the substrate 15 that will be described later below, between the light emitting element 11a1 and the light emitting element 11b1. Similarly, in order to receive light emitted by the light emitting element 11a2 and the light emitting element 11b2, the light receiving element 12-2 is arranged, on the upper surface 15a of the substrate 15 that will be described later below, between the light emitting element 11a2 and the light emitting element 11b2.

The control part 13 is constituted by a microcomputer. The control part 13 performs control to transmit a timing signal to the driving circuit 11c of the light emitting part 11 to emit near-infrared light from the light emitting element package 11a and the light emitting element package 11b of the light emitting part 11. Specifically, the control part 13 causes the light emitting element 11a1 of the light emitting element package 11a and the light emitting element 11b1 of the light emitting element package 11b to simultaneously emit near-infrared light and to stop emitting the light after a predetermined period of time. Subsequently, the control part 13 causes the light emitting element 11a2 of the light emitting element package 11a and the light emitting element 11b2 of the light emitting element package 11b to simultaneously emit near-infrared light and to stop emitting the light after a predetermined period of time. Thereby, near-infrared light of the central wavelength λ1 and near-infrared light of the central wavelength λ2 are emitted intermittently and alternately.

Further, the control part 13 uses an internal analog-to-digital converter circuit to convert amplified light reception signals output from the amplifier circuit 12b of the light receiving part 12 into processable digital signal information (signal output values), and estimates, based on the converted signal information, respective biological information about a hemoglobin change in blood, an oxygen rate change in blood, and a pulse rate. The control part 13 serves as a biological information estimating part.

The wireless communication part 14 is constituted by a wireless communication IC. The biological information estimated by the control part 13 is transmitted to the display device 20 that will be described later below through communication using a wireless comunication standard such as Bluetooth (registered trademark), for example. Note that the sensor module 10 may have a configuration that transmits, to the display device 20, signal information used to estimate biological information as described above through wireless communication instead of transmitting biological information such that the biological information may be estimated based on the signal information in the display device 20.

The substrate 15 is a printed circuit board on which a wiring pattern is formed with a copper foil on a glass epoxy substrate. As illustrated in FIG. 4, on the upper surface 15a of the substrate 15, the light emitting element package 11a including the light emitting element 11a1 and the light emitting element 11a2, the light emitting element package 11b including the light emitting element 11b1 and the light emitting element 11b2, and the light receiving element package 12a including the light receiving element 12-1 and the light receiving element 12-2 are mounted. On the upper surface 15a, the light emitting element 11a1 and the light emitting element 11b1 are arranged at an interval, and the light receiving element 12-1 is arranged at an intermediate position between the light emitting element 11a1 and the light emitting element 11b1. The light emitting element 11a1, the light emitting element 11b1, and the light receiving element 12-1 are arranged on one straight line. According to the present embodiment, each of the interval L1 between the light emitting element 11a1 and the light receiving element 12-1 and the interval L2 between the light emitting element 11b1 and the light receiving element 12-1 is 4 mm. It is preferable that the interval L1 and the interval L2 are in a range of from 4 mm to 11 mm. Further, it is preferable that the interval L1 is the same as the interval L1. The light emitting element 11a2, the light emitting element 11b2, and the light receiving element 12-2 are arranged similar to the above arrangement. On the non-illustrated lower surface of the substrate 15, the driving circuit 11c of the light emitting part 11, the amplifier circuit 12b of the light receiving part 12, the microcomputer constituting the control part 13, and the wireless communication IC constituting the wireless communication part 14 are mounted.

As illustrated in FIG. 3, the case 18 is formed into a shape of hollow box, an upper wall 18a of the case 18 has translucency, and parts other than the upper wall 18a are constituted by a material having a light-blocking property. In the case 18, the substrate unit 16 is accommodated such that the upper surface 15a of the substrate 15 faces the upper wall 18a. The case 18 is attached to an object under detection such that the upper wall 18a contacts the surface of the object under detection (skin of a person). In this way, the light emitting element 11a1 and the light emitting element 11a2 of the light emitting element package 11a, the light emitting element 11b1 and the light emitting element 11b2 of the light emitting element package 11b, and the light receiving element 12-1 and the light receiving element 12-2 of the light receiving element package 12a are located to face the surface of the object under detection via the upper wall 18a.

The display device 20 is a tablet terminal and is able to execute various types of application programs (which are simply referred to as "applications" hereinafter) such as applications downloaded in advance and applications downloaded from the Internet. Thus, the display device 20 serves as various devices by executing an application in accordance with a purpose. According to the present embodiment, the display device 20 serves as a device that constitutes a part of the biological information displaying system 1 by executing an application for displaying biological information in the display device 20.

As illustrated in FIG. 2, the display device 20 includes a display part 21 made of a liquid crystal display, a touch panel 22 stacked on the surface of the liquid crystal display, a control part 23 including a microcomputer, a storage part 24 including a working memory and a memory for storing information, and a wireless communication part 25 made of a wireless communication module.

The display part 21 displays various screens such as documents and images in accordance with display control information output from the control part 23 and displays, within the various screens, operation items such as buttons, text input areas, a keyboard, and a numeric keypad.

To the touch panel 22, a contact operation is input by a user at a location corresponding to an above described operation item. The touch panel 22 outputs, to the control part 23, a signal in accordance with the input contact operation.

For example, information relating to the contact information input by the user to the touch panel 22 is input to the control part 23, and the control part 23 outputs, based on the input information, display control information for displaying a predetermined image to the display part 21. Further, various kinds of information are transmitted and received between the control part 23 and the storage part 24. The control part 23 reads predetermined information from the storage part 24, and causes the storage part 24 to store predetermined information. The wireless communication part 25 receives biological information transmitted from the sensor module 10, and the control unit 23 takes in the biological information received by the wireless communication part 25.

According to the present embodiment, the display device 20 is able to execute a biological information dislpaying application program as a biological information dislpaying program for displaying biological information transmitted from the sensor module 10 (in the following, referred to as the "biological application" simply).

In the biological information displaying system 1, the sensor module 10 starts a biological information estimating operation upon power being supplied by operating a non-illustrated power switch. As the biological information estimating operation, the sensor module 10 emits near-infrared light from the light emitting part 11, receives, by the light receiving part 12, near-infrared light that has passed through an objec under detection, estimates, based on the received near-infrared light, respective biological informtion about a hemoglobin change in blood, an oxygen rate change in blood, and a pulse rate, and successively transmits the respective biological information to the display device 20. The display device 20 causes the control part 23 to execute the biological application to display, on the display part 21, the biological information transmitted from the sensor module 10.

Specifically, the biological information is estimated as follows. Receiving a control signal from the control part 13, the driving circuit 11c causes the light emitting element 11a1 and the light emitting element 11b1, which emit near-infrared light of which the central wavelength λ1 is 760 mn, and the light emitting element 11a2 and the light emitting element 11b2, which emit near-infrared light of which the central wavelength λ2 is 850 mn, to alternately emit light to an object under detection at predetermined timings. Then, each of the light receiving element 12-1 and the light receiving element 12-2 receives weak reflection light reflected by the object under detection. The light receiving element 12-1 and the light receiving element 12-2 output signals in accordance with the received reflection light, and the signals are amplified by the amplifier circuit 12b and the amplified signals are input to the control part 13. The control part 13 converts the input signals from analog to digital and obtains signal outputs for the respective wavelengths (which are 760 nm and 850 nm). In the control part 13, a calculation formula or a table that represents a relationship between these signal output values and values of biological information is stored in advance in a memory or the like. By referring to this, respective biological information in accordance with the signal output values are obtained.

Next, effects of the present invention will be described with reference to FIG. 5 to FIG. 9. FIG. 5 is a graph illustrating, for each of a configuration including two light emitting elements constituting a pair and a configuration including only one light emitting element, a relationship between detection sensitivity and a planar distance from a reactive member.

For the above described sensor module 10, a configuration was made as working example 1 in which the light emitting element 11a2, the light emitting element 11b2, and the light receiving element 12-2 are omitted and two light emitting elements 11a1 and 11b1 that consitutute a pair between which a light receiving element 12-1 is arranged are provided. With respect to the configuration of this working example 1, a configuration was made as comparative example 1 in which the light emitting element 11b1 is omitted and a single light emitting element 11a1 and a single light receiving element are provided. Then, with respect to each of working example 1 and comparative example 1, the light emitting element 11a1 was caused to emit light to a simulated object under detection at a predetermined timing (with respect to working example 1, the light emitting element 11b1 was also caused to emit light at the same time), and a signal output by the light receiving element 12-1 was measured while shifting, in the arrangement direction of the light emitting element 11a1 and the light receiving element 12-1, the surface of the simulated object under detection into which a member simultating an artery was embedded (referred to as the "reactive member", hereinafter). In working example 1 and comparative example 1, the interval between the light emitting element 11a1 and the light receiving element 12-1 was 4 mm. Further, in working example 1, the interval between the light emitting element 11b1 and the light receiving element 12-1 was also 4 mm. For the light emitting element 11a1 and the light emitting element 11b1, elements that separately and alternately emit two types of near-infrared light that are near-infrared light whose central wavelength λ1 is 760 nm and near-infrared light whose central wavelength λ2 is 850 nm were used. For the light receiving element 12-1, an element that is senstive to both near-infrared light close to the central wavelength λ1 and near-infrared light close to the central wavelength λ2 was used. The measured values are plotted in the graph illustrated in FIG. 5. In FIG. 5, the horizontal axis represents a relative planar distance [mm] between the reactive member and the light receiving element, and the vertical axis represents a signal output value (in a suitable unit) obtained by analog-to-digital converting a signal output by the amplifier circuit 12b. This signal output value is normalized by a driving current of the light emitting element. It indicates that as the signal output value increases, the detection sensitivity increases. Further, in FIG. 5, the continuous line indicates working example 1 and the broken line indicates comparative example 1.

As illustrated in FIG. 5, in comparative example 1, the detection sensitivity is the highest when the reactive member is at the intermediate position between the light emitting element 11a1 and the light receiving element 12-1 (the position at which the relative planar distance is -2 mm), and the detection sensitivity decreases upon the reactive member being away from the intermediate position. In contrast, in working example 1, when the relative planar distance from the reactive member is in a range of from -2 mm to 4 mm, the detection sensitivity is substantially constant and is higher than that in comparative example 1, and the detection sensitivity decreases upon the relative planar distance being away from this range.

That is, by working example 1 having a configuration in which the two light emitting elements are provided, it is found that a higher detection sensitivity can be obtained in a wider range in the planar direction relative to comparative example 1 in which only one light emitting element is provided. Further, by obtaining a high detection sensitivity, it is found that biological information can be obtained even when an artery of an object under detection is at a deeper position.

The graph for the above described working example 1 is equivalent to that obtained by combining graphs of detection sensitivity independently measured for respective two light emitting elements. This allows predicting detection sensitivity of a configuration in which two light emitting elements are provided, by measuring detection sensitivity for a configuration in which one light emitting element is provided.

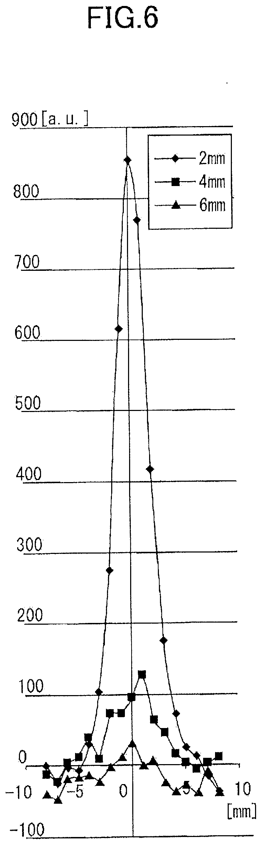

In consideration of the above, using a configuration in which the light emitting element 11a2, the light emitting element 11b2, and the light receiving element 12-2 are omitted, the light emitting element 11b1 that is one of the two light emitting elements 11a1 and 11b1 constituting a pair is omitted, and one light emitting element 11a1 and one light receiving element 12-1 are provided for the above described sensor module 10, relationships between intervals between the light emitting element 11a1 and the light receiving element 12-1 and detection sensitivities in a depth direction were measured. The measured results are illustrated in FIG. 6 to FIG. 9.

FIG. 6 to FIG. 9 are graphs illustrating, for configurations in which the intervals between the light emitting element 11a1 and the light receiving element 12-1 were 2.5 mm, 4 mm, 7 mm, and 10 mm, relationships between detection sensitivities and relative distances from a reactive member.

For FIG. 6, the interval between the light emitting element 11a1 and the light receiving element 12-1 was 2.5 mm, the light emitting element 11a1 was caused to emit light at a predetermined timing to a simulated object under detection at a driving current 0.4 mA and a signal output by the light receiving element 12-1 was measured while shifting, in the arrangement direction of the light emitting element 11a1 and the light receiving element 12-1, the surface of the simulated object under detection into which a reactive member was embedded. For the light emitting element 11a1, an element that separately and alternately emits two types of near-infrared light that are near-infrared light whose central wavelength λ1 is 760 nm and near-infrared light whose central wavelength λ2 is 850 nm was used. For the light receiving element 12-1, an element that is senstive to both near-infrared light close to the central wavelength λ1 and near-infrared light close to the central wavelength λ2 was used. For the simulated objects under detection, samples were prepared into which a reactive member was embedded at positions at which the depths from its surface were 2 mm, 4 mm, and 6 mm. In FIG. 6, the horizontal axis represents a relative planar distance [mm] between the reactive member and the light receiving element, and the vertical axis represents a signal output value (in a suitable unit) obtained by analog-to-digital converting a signal output by the amplifier circuit 12b. This signal output value is normalized by a driving current of the light emitting element. It indicates that as the signal output value increases, the detection sensitivity increases. Further, in FIG. 6, the graphs indicated by the diamonds (◆), the squares (■), and the triangles (▲) indicate measurement results when depths of the reactive members were 2 mm, 4 mm and 6 mm, respectively.

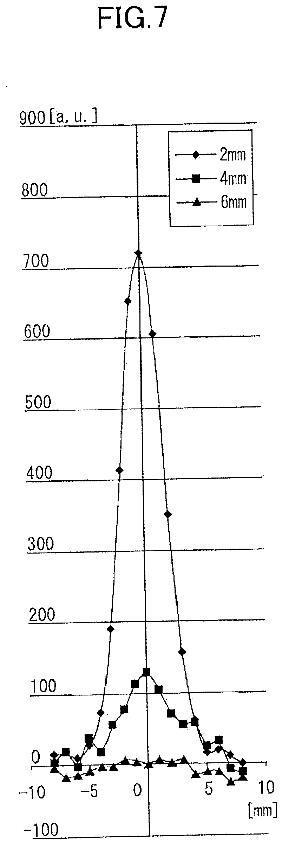

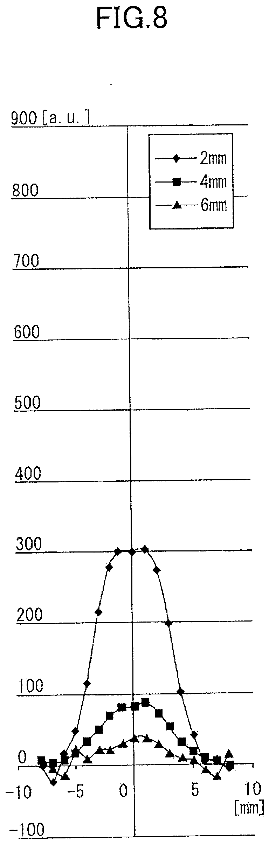

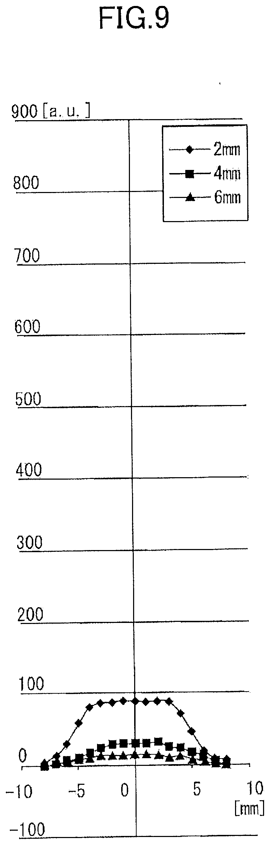

For FIG. 7, measurement was performed by a method similar to the measurement method of FIG. 6 except that the interval between the light emitting element 11a1 and the light receiving element 12-1 was 4 mm and the driving current of the light emitting element 11a1 was 1 mA. For FIG. 8, measurement was performed by a method similar to the measurement method of FIG. 6 except that the interval between the light emitting element 11a1 and the light receiving element 12-1 was 7 mm and the driving current of the light emitting element 11a1 was 3 mA. For FIG. 9, measurement was performed by a method similar to the measurement method of FIG. 6 except that the interval between the light emitting element 11a1 and the light receiving element 12-1 was 10 mm and the driving current of the light emitting element 11a1 was 10 mA.

For FIG. 6 to FIG. 9, the interval between the light emitting element 11a1 and the light receiving element 12-1 is sequentially increased from 2.5 mm to 10 mm. Then, the graphs of the detection sensitivity with respect to the reactive member located at the deepest position (of depth 6 mm) are focused on. In the graph of FIG. 6 for which the interval is the smallest, the reactive member cannot be detected because of large noize. In contrast, the graphs of FIG. 7 to FIG. 9 for which the intervals are relatively large have graph shpaes in accordance with the reactive members, and the reactive members can be detected. In particular, by the configuration in which the interval between the light emitting element 11a1 and the light receiving element 12-1 is 7 mm, a relatively high detection sensitivity is obtained. It is found that the detection sensitivity decreases as the interval is decreased or increased from 7 mm.

That is, it is found that a reactive member at a deep position can be detected in a configuration in which the interval between the light emitting element 11a1 and the light receiving element 12-1 is approximately in a range of from 4 mm to 11 mm.

Therefore, by providing a plurality of light emitting elements and making an interval between the light emitting elements and a light receiving element an appropriate distance, a part under detection such as an artery located in a wide range in the surface direction and the depth direction of the object under detection can be detected. For example, a large effect can be obtained in particluar when wearing it on a wrist or the like of a person to measure a part under detection having a large individual difference in position such as a radial (artery) in the wrist located at a relatively deep location.

Therefore, according to the sensor module 10 and the biological information displaying system 1 including the sensor module 10 according to present the embodiment, positional adjustment onto a position at which biological information can be obtained is facilitated when being attached to an object under detection.

Although the sensor module 10 has a configuration that includes the light emitting element 11a1, the light emitting element 11b1, the light receiving element 12-1, the light emitting element 11a2, the light emitting element 11b2, and the light receiving element 12-2 in the embodiment described above, the sensor module 10 is not limited to this. For example, the sensor module 10 may have a configuration in which the light emitting element 11a2, the light emitting element 11b2, and the light receiving element 12-2 illustrated in FIG. 4 are omitted and only the light emitting element 11a1, the light emitting element 11b1, and the light receiving element 12-1 are provided. In this configuration, the light emitting element 11a1 and the light emitting element 11b1 may emit only near-infrared light having the same central wavelength λ1 or may separately emit both near-infrared light having the central wavelength λ1 and near-infrared light having the central wavelength λ2.

Further, as illustrated in FIG. 4, the light emitting element 11a1 and the light emitting element 11a2 are arranged side by side in the vertical direction such that the distance between the light emitting element 11a1 and the light receiving element 12-1 is equal to the distance between the light emitting element 11a2 and the light receiving element 12-2. Other than this, the light emitting element 11a1 and the light emitting element 11a2 may be arranged on one straight line side by side in the horizontal direction of FIG. 4, and the light emitting element 11b1 and the light emitting element 11b2 may also be similarly arranged on the straight line.

Further, the embodiment described above has a configuration in which a wavelength at which the receiving sensitivity of a light receiving element is the maximum is in accordance with a central wavelength of near-infrared light emitted by a light emitting element corresponding to the light receiving element. However, when the light receiving element can detect near-infrared light emitted by the light emitting element, the wavelength at which the receiving sensitivity of the light receiving element is the maximum may differ somewhat from the central wavelength of the light emitting element. Further, an embodiment may have a configuration in which a single light receiving element receives a plurality of kinds of near-infrared light having differing central wavelengths. Further, an embodiment may have a configuration in which a single light emitting element emits a plurality of kinds of near-infrared light having differing central wavelengths.

Note that although the embodiments and its applied examples have been described above, the present invention is not limited to these examples. With respect to the embodiments and its applied examples described above, a range of the present invention may allow a person skilled in the art to add, remove, or change an element and to combine characteristics of the embodiments without departing from the scope of the present invention.

### Explanation of reference numerals

- 1: biological information displaying system
- 10: sensor module
- 11: light emitting part
- 11a, 11b: light emitting element package
- 11a1, 11a2, 11b1, 11b2: light emitting element
- 11c: driving circuit
- 12: light receiving part
- 12a: light receiving element package
- 12-1, 12-2: light receiving element
- 12b: amplifier circuit
- 13: control part
- 14: wireless communication part
- 15: substrate
- 15a: upper surface (of substrate)
- 16: substrate unit
- 18: case
- 18a: upper wall (of case)
- 20: display device
- 21: display part
- 22: touch panel
- 23: control part
- 24: storage part
- 25: wireless communication part

## Claims

1. A sensor module for estimating biological information comprising:
a light emitting part configured to emit light including near-infrared light to an object under detection; and
a light receiving part configured to receive the light that has passed through the object under detection,
wherein the sensor module generates a signal in accordance with near-infrared light included in the light received by the light receiving part,
wherein the light emitting part includes a plurality of light emitting elements arranged at an interval,
wherein the light receiving part includes one or more light receiving elements that are sensitive to near-infrared light,
wherein the one or more light receiving elements are arranged to receive the light emitted by each of the plurality of light emitting elements, and
wherein each of the plurality of light emitting elements emits near-infrared light having a same central wavelength.

2. The sensor module according to claim 1, wherein the light receiving elements are arranged between two light emitting elements included in the plurality of light emitting elements.

3. The sensor module according to claim 1 or 2, wherein the light receiving elements are arranged with the plurality of light emitting elements at an interval of from 4 mm to 11 mm.

4. The sensor module according to any one of claims 1 to 3, wherein the light emitting part further includes a plurality of other light emitting elements each of which emits near-infrared light having another same central wavelength, which is different from the central wavelength of the near-infrared light emitted by the plurality of light emitting elements.

5. The sensor module according to any one of claims 1 to 3, wherein each of the plurality of light emitting elements emits near-infrared light having the central wavelength and near-infrared light having another same central wavelength, which is different from the central wavelength.

6. The sensor module according to claim 4 or 5, wherein the central wavelength is shorter than 805 nm, and said another central wavelength is longer than 805 nm.

7. The sensor module according to claim 6, wherein the central wavelength is 760 nm, and said another central wavelength is 850 nm.

8. The sensor module according to any one of claims 1 to 3,
wherein the light emitting part further includes a plurality of other light emitting elements each of which emits near-infrared light having another same central wavelength, which is different from the central wavelength of the near-infrared light emitted by the plurality of light emitting elements, and
wherein said another central wavelength is 640 nm, and the central wavelength is 940 nm.

9. The sensor module according to any one of claims 1 to 8, wherein a wavelength of light at which receiving sensitivity in the one or more light receiving elements is highest is the central wavelength.

10. The sensor module according to any one of claims 4 to 7,
wherein the light receiving part further includes one or more other light receiving elements that are sensitive to near-infrared light,
wherein the one or more other light receiving elements are arranged to receive the light emitted by each of the plurality of other light emitting elements,
wherein a wavelength of light at which receiving sensitivity in the one or more light receiving elements is highest is the central wavelength, and
wherein a wavelength of light at which receiving sensitivity in the one or more other light receiving elements is highest is said another central wavelength.

11. A biological information displaying system comprising:
the sensor module according to any one of claims 1 to 10;
a biological information estimating part configured to estimate biological information based on the signal generated by the receiving part; and
a display part configured to display the biological information estimated by the biological information estimating part.

12. The biological information displaying system according to claim 11, wherein the biological information estimating part estimates, as the biological information, at least one selected from a hemoglobin change in blood, an oxygen rate change in blood, and a pulse rate.
